# EUROPÄISCHE PATENTSCHRIFT

(11) **EP 0 722 992 B1**
(45) Veröffentlichungstag und Bekanntmachung des Hinweises auf die Patenterteilung: **20.02.2008**
(21) Anmeldenummer: 95120194.6
(22) Anmeldetag: 20.12.1995
(51) Int. Cl.: C09C 3/12, C01B 13/22, C09C 3/00, C08K 9/06, G03G 9/00, A61K 8/00

(54) **Oberflächenmodifizierte pyrogen hergestellte Mischoxide, Verfahren zu ihrer Herstellung und Verwendung**
Surface modified pyrogenic mixed oxide, process for its preparation and its use
Oxyde mixte pyrogénique modifié en surface, procédé pour sa préparation et son utilisation

(30) Priorität: 12.01.1995 DE 19500674
(43) Veröffentlichungstag der Anmeldung: 24.07.1996
(73) Patentinhaber: Evonik Degussa GmbH, 40474 Düsseldorf (DE)
(72) Erfinder: Ettlinger, Manfred, Dr., D-63791 Karlstein (DE); Hartmann, Werner, Dr., D-64832 Babenhausen (DE); Kerner, Dieter, Dr., Nordland Park, New Jersey 07432 (US); Meyer, Jürgen, Dr., D-79618 Rheinfelden (DE)

(56) Entgegenhaltungen:
- EP-A- 0 373 426
- DD-A- 33 175
- DE-A- 1 916 360
- DE-A- 3 707 226
- FR-A- 2 196 376
- GB-A- 1 031 764

## Beschreibung

Die Erfindung betrifft oberflächenmodifizierte pyrogen hergestellte Mischoxide, das Verfahren zu ihrer Herstellung und deren Verwendung.

Gegenstand der Erfindung sind oberflächenmodifizierte pyrogen hergestellte Mischoxide, die zwei oder mehr Komponenten aus der Reihe SiO₂, Al₂O₃, TiO₂, ZrO₂, Fe₂O₃, Nb₂O₅, V₂O₅, WO₃, SnO₂, GeO₂ enthalten und die von anhaftenden Chlorwasserstoff in feuchter Luft befreit wurden und mit einer oder mehreren Verbindungen aus den folgenden Gruppen oberflächenmodifiziert wurden:
(a) Organosilane des Types (RO)₃Si(CₙH₂ₙ₊₁)
   R = Alkyl, wie z. B. Methyl-, Ethyl-, n-Propyl-, i-Propyl-, Butyl-
   n = 1 - 20
(b) Organosilane des Types R'ₓ(RO)_{y}Si(CₙH₂ₙ₊₁)
   R = Alkyl, wie z. B. Methyl-, Ethyl-, n-Propyl-, i-Propyl-, Butyl-
   R' = Alkyl, wie z. B. Methyl-, Ethyl-, n-Propyl-, i-Propyl-, Butyl-
   n = 1 - 20
   x+y = 3
   x = 1,2
   y = 1,2
(f) Organosilane des Types (RO)₃Si(CH₂)ₘ-R'
   R = Alkyl, wie Methyl-, Ethyl-, Propyl-
   m = 0,1 - 20
   R' = Methyl-, Aryl (z.B. -C₆H₅, substituierte Phenylreste) -C₄F₉, OCF₂-CHF-CF₃, -C₆F₁₃, -O-CF₂-CHF₂
   -NH₂, -N₃, -SCN, -CH=CH₂,
   -OOC(CH₃)C = CH₂
   -OCH₂-CH(O)CH₂ 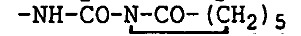 -NH-COO-CH₃, -NH-COO-CH₂-CH₃, -NH-(CH₂)₃Si(OR)₃ -Sₓ-(CH₂)₃Si(OR)₃
(g) Organosilane des Typs (R")ₓ(RO)_{y}Si(CH₂)ₘ-R'
   R" = Alkyl x+y = 3
   x = 1,2
   y = 1,2
   m = 0,1 bis 20
   R' = Methyl-, Aryl (z.B. -C₆H₅ ,substituierte Phenylreste) -C₄F₉, -OCF₂-CHF-CF₃, -C₆F₁₃, -O-CF₂-CHF₂ -NH₂, -N₃, -SCN, -CH=CH₂, -OOC(CH₃)C = CH₂ -OCH₂-CH(O)CH₂ 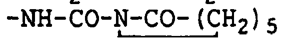 -NH-COO-CH₃, -NH-COO-CH₂-CH₃, -NH-(CH₂)₃Si(OR)₃ -Sₓ-(CH₂)₃Si(OR)₃
   R = Methyl-, Ethyl-
(k) Silazane des Types 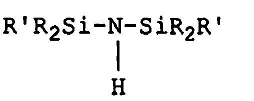 R = Alkyl
   R' = Alkyl, Vinyl
(m) Polysiloxane bzw. Silikonöle des Types 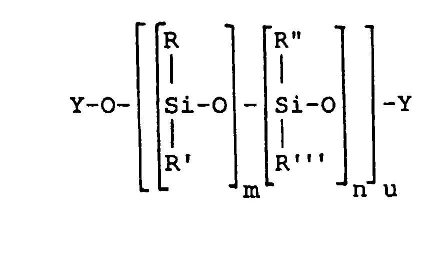 m = 0,1,2,3,...∞
   n = 0,1,2,3,...∞
   u = 0,1,2,3,...∞
   Y=CH₃, H, CₙH₂ₙ₊₁ n=1-20
   Y=Si(CH₃)₃, Si(CH₃)₂H
   Si(CH₃)₂OH, Si(CH₃)₂(OCH₃)
   Si (CH₃)₂ (CₙH₂ₙ₊₁) n=1-20
   R = Alkyl, wie CₙH₂ₙ₊₁, wobei n = 1 bis 20 ist, Aryl, wie Phenyl- und substituierte Phenylreste, (CH₂)ₙ-NH₂, H
   R' = Alkyl, wie CₙH₂ₙ₊₁, wobei n = 1 bis 20 ist, Aryl, wie Phenyl- und substituierte Phenylreste, (CH₂)ₙ-NH₂, H
   R'' = Alkyl, wie CₙH₂ₙ₊₁, wobei n = 1 bis 20 ist, Aryl, wie Phenyl- und substituierte Phenylreste, (CH₂)ₙ-NH₂, H
   R''' = Alkyl, wie CₙH₂ₙ₊₁, wobei n = 1 bis 20 ist, Aryl, wie Phenyl- und substituierte Phenylreste, (CH₂)ₙ-NH₂, H

Ein weiterer Gegenstand der Erfindung ist ein Verfahren zur Herstellung der erfindungsgemäßen oberflächenmodifizierten pyrogen hergestellten Mischoxide, welches dadurch gekennzeichnet ist, daß man zwei oder mehr Metallchloride aus der Reihe SiCl₄, AlCl₃, TiCl₄, ZrCl₄, FeCl₃, NbCl₅, VOCl₃, WOCl₄, WCl₆, SnCl₄ und GeCl₄ gemeinsam oder getrennt verdampft, zusammen mit einem Inertgas, zum Beispiel Stickstoff, in die Mischkammer eines bekannten Brenners überführt, dort mit Wasserstoff, Luft und/oder Sauerstoff vermischt, das Mehrkomponentengemisch in einer Reaktionskammer verbrennt, danach die festen Mischoxide von den gasförmigen Reaktionsprodukten abtrennt und in feuchter Luft von anhaftendem Chlorwasserstoff befreit und die pyrogen hergestellten Mischoxide in einem geeigneten Mischgefäß vorlegt, unter intensivem Mischen die Mischoxide gegebenenfalls zunächst mit Wasser und anschließend mit dem Oberflächenmodifizierungsreagenz oder dem Gemisch von mehreren Oberflächenmodifizierungsreagentien besprüht, 15 bis 30 Minuten nachmischt und anschließend bei einer Temperatur von 100 bis 400 °C über einen Zeitraum von 1 bis 6 Stunden tempert.

Das eingesetzte Wasser kann mit einer Säure, zum Beispiel Salzsäure, bis zu einem pH-Wert von 7 bis 1 angesäuert sein. Das eingesetzte Oberflächenmodifizierungsreagenz kann in einem geeigneten Lösungsmittel, wie zum Beispiel Ethanol, gelöst sein. Das Mischen und/oder die Temperung kann in einer Schutzgasatmosphäre, wie zum Beispiel Stickstoff, durchgeführt werden.

Ein weiterer Gegenstand der Erfindung ist die Verwendung des erfindungsgemäßen oberflächenmodifizierten pyrogen hergestellten Mischoxides als
- Verstärkerfüllstoff in Silikonkautschuk und Gummi
- Ladungsstabilisator und Rieselhilfsmittel in Tonerpulver
- Rieselhilfsmittel (free-flow-agent)
- Antiblockingmittel, zum Beispiel in Folien
- UV-Blocker, zum Beispiel in Kosmetika
- Verdickungsmittel, zum Beispiel in Lacken

### Beispiele

### Beispiele für die Herstellung von Mischoxiden

Die Metallchloride 1 und 2 werden in getrennten Verdampfern verflüchtigt und die Chloriddämpfe mittels Stickstoff in die Mischkammer eines Brenners geleitet. Dort werden sie mit Wasserstoff und getrockneter Luft und/oder Sauerstoff vermischt und in einer Reaktionskammer verbrannt. In der Koagulationsstrecke werden die Reaktionsprodukte auf etwa 110 °C abgekühlt und die entstandenen Mischoxide anschließend mit einem Filter abgeschieden. Durch eine Behandlung der Pulver mit feuchter Luft bei Temperaturen zwischen 500 und 700 °C wird anhaftendes Chlorid entfernt.

Die Reaktionsbedingungen und die Produkteigenschaften für die verschiedenen Mischoxide sind in Tabelle 1 zusammengestellt.

### Beispiele für die Herstellung von oberflächenmodifizierten Mischoxiden

Die Reaktionsbedingungen und stöchiometrischen Verhältnisse für die Oberflächenmodifizierung können der Tabelle 2 entnommen werden. Die physikalisch-chemischen Daten sind in Tabelle 3 zusammengefaßt.

**Tabelle 1: Reaktionsbedingungen und Produkteigenschaften einiger Mischoxide**

| **Mischoxid Beispiel** | **Oxid 1** | **Oxid 2** | **Metall-chlorid 1** | **Metall-chlorid 2** | **H₂** | **Luft** | **BET** | **Stampf-dichte** | **Glüh-verlust** | **Chlorid-gehalt** |
|---|---|---|---|---|---|---|---|---|---|---|
| | **[G. %]** | **[G. %]** | **[g/h]** | **[g/h]** | **[l/h]** | **[l/h]** | **[m²/g]** | **[g/l]** | **[%]** | **[%]** |
| 1 | SiO₂ 7,5 | TiO₂ 92,3 | SiCl₄ 50 | TiCl₄ 516 | 470 | 3280 | 95 | 85 | 0,6 | 0,1 |
| 2 | Al₂O₃ 15 | SiO₂ 85 | AlCl₃ 126 | SiCl₄ 778 | 300 | 1300 | 179 | 104 | 2,9 | 0,12 |
| 3 | Fe₂O₃ 2 | TiO₂ 98 | FeCl₃ 29 | TiCl₄ 1697 | 525 | 3079 | 53 | 175 | 1,2 | 0,3 |
| 4 | Fe₂O₃ 7 | TiO₂ 93 | FeCl₃ 107 | TiCl₄ 1613 | 525 | 3079 | 46 | 185 | 1,7 | 0,4 |
| 5 | SiO₂ 87 | ZrO₂ 13 | SiCl₄ 1303 | ZrCl₄ 135 | 800 | 2420 | 121 | 48 | 0,9 | 0,24 |
| 6 | Al₂O₃ 11 | TiO₂ 89 | AlCl₃ 188 | TiCl₄ 793 | 448 | 1276 | 47 | 329 | 0,7 | 0,16 |
| 7 | Al₂O₃ 25 | TiO₂ 75 | AlCl₃ 464 | TiCl₄ 1269 | 525 | 3579 | 72 | 120 | 1,1 | 0,6 |
| 8 | TiO₂ 95 | ZrO₂ 5 | TiCl₄ 1661 | ZrCl₄ 78 | 525 | 3080 | 59 | 218 | 0,9 | 0,15 |

**Tabelle 2**

| **Beispiel** | **Bezeich-nung** | **Mischoxid** | **Modifi-zierungsreagenz*** | **Modifizierungsreagenz** | **Wassermenge** | **Lösungsmittelmenge** | **Temperzeit** | **Temper temperatur** |
|---|---|---|---|---|---|---|---|---|
| | | | | **[g/100 g Mischer** | **[g/100 g Mischoxid]** | **[g/100 g Mischoxid]** | **[h]** | **[°C]** |
| 9 | VT 772 | 4 | 1 | 10 | 0 | 0 | 4 | 140 |
| 10 | VT 773 | 4 | 2 | 10 | 0 | 0 | 2 | 120 |
| 11 | VT 774 | 4 | 3 | 10 | 0 | 0 | 2,5 | 250 |
| 12 | VT 816 | 3 | 1 | 10 | 0 | 0 | 3 | 180 |
| 13 | VT 817 | 3 | 2 | 10 | 0 | 0 | 2 | 120 |
| 14 | VT 818 | 3 | 3 | 10 | 0 | 0 | 2,5 | 250 |
| 15 | VT 775 | 2 | 1 | 20 | 0 | 0 | 4 | 140 |
| 16 | VT 776 | 2 | 2 | 16 | 0 | 0 | 2 | 120 |
| 17 | VT 777 | 2 | 3 | 15 | 0 | 0 | 2 | 250 |
| 18 | VT 819 | 8 | 1 | 10 | 0 | 0 | 4 | 180 |
| 19 | VT 820 | 8 | 2 | 10 | 0 | 0 | 2 | 120 |
| 20 | VT 821 | 8 | 3 | 10 | 0 | 0 | 2,5 | 250 |
| 21 | VT 900 | 4 | 2 | 12 | 5 | 0 | 2,5 | 140 |
| 22 | VT 901 | 3 | 2 | 10 | 0 | 10** | 2,5 | 140 |
| 23 | VT 797 | 6 | 2 | 5 | 0 | 0 | 2 | 120 |
| 24 | VT 748 | 6 | 2 | 10 | 0 | 0 | 2 | 120 |
| 25 | VT 749 | 6 | 4 | 10 | 0 | 0 | 2 | 120 |
| 26 | VT 750 | 7 | 2 | 10 | 0 | 0 | 2 | 120 |
| 27 | VT 751 | 7 | 4 | 10 | 0 | 0 | 2 | 120 |
| 28 | VT 719 | 5 | 1 | 10 | 5 | 0 | 3 | 130 |
| 29 | VT 734 | 1 | 3 | 10 | 0 | 0 | 2 | 250 |

| | | | | | | | | |
|---|---|---|---|---|---|---|---|---|
| * 1 = Hexamethyldisiliazan = (CH₃)₃Si-NH-Si(CH₃)₃ 2 = Trimethoxy-octyl-silan = (CH₃O)₃Si-(CH₂)₇-CH₃ 3 = Dimethylpolysiloxan = 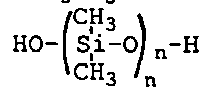 4 = Trimethoxy-propyl-silan = (CH₃O)₃Si-CH₂-CH₂-CH₃ ** = Ethanol | | | | | | | | |

**Tabelle 3**

| **Beispiel** | **Bezeichnung** | **Spezifische Oberfläche nach BET** | **Stampdichte** | **Trocknungsverlust** | **Glüverlust** | **pH-Wert** | **Kohlenstoffgehalt** |
|---|---|---|---|---|---|---|---|
| | | **[m^{/2}/g]** | | **[g/l]** | **%** | | **[%]** |
| 9 | VT 772 | 40 | 269 | 0,0 | 1,3 | 6,4 | 0,5 |
| 10 | VT 773 | 36 | 280 | 0,1 | 4,7 | 3,4 | 3,6 |
| 11 | VT 774 | 27 | 301 | 0,2 | 3,9 | 3,4 | 2,7 |
| 12 | VT 816 | 45 | 258 | 0,4 | 1,1 | 7,5 | 0,5 |
| 13 | VT 817 | 39 | 288 | 0,7 | 3,9 | 3,4 | 3,5 |
| 14 | VT 818 | 32 | 292 | 0,0 | 3,6 | 3,6 | 2,9 |
| 15 | VT 775 | 124 | 127 | 0,5 | 3,4 | 6,6 | 1,7 |
| 16 | VT 776 | 111 | 136 | 1,0 | 9,5 | 4,2 | 5,8 |
| 17 | VT 777 | 101 | 136 | 0,9 | 4,7 | 4,2 | 2,9 |
| 18 | VT 819 | 51 | 345 | 0,5 | 0,7 | 9,0 | 0,4 |
| 19 | VT 820 | 45 | 275 | 0,0 | 4,4 | 4,0 | 3,6 |
| 20 | VT 821 | 35 | 275 | 0,0 | 2,3 | 4,1 | 2,5 |
| 21 | VT 900 | 34 | 275 | 0,1 | 4,9 | 3,5 | 3,9 |
| 22 | VT 901 | 38 | 282 | 0,6 | 4,0 | 3,6 | 3,6 |
| 23 | VT 797 | 31 | 396 | 0,2 | 1,7 | 3,7 | 2,0 |
| 24 | VT 748 | 23 | 409 | 0,3 | 4,8 | 4,0 | 3,9 |
| 25 | VT 749 | 26 | 402 | 0,3 | 2,3 | 4,1 | 1,8 |
| 26 | VT 750 | 56 | 161 | 0,3 | 16,8 | 3,8 | 3,9 |
| 27 | VT 751 | 55 | 162 | 0,2 | 2,7 | 4,1 | 2,0 |
| 28 | VT 719 | 60 | 60 | 0,1 | 1,1 | 6,4 | 1,3 |
| 29 | VT 734 | 74 | 114 | 0,5 | 2,5 | 4,2 | 2,5 |

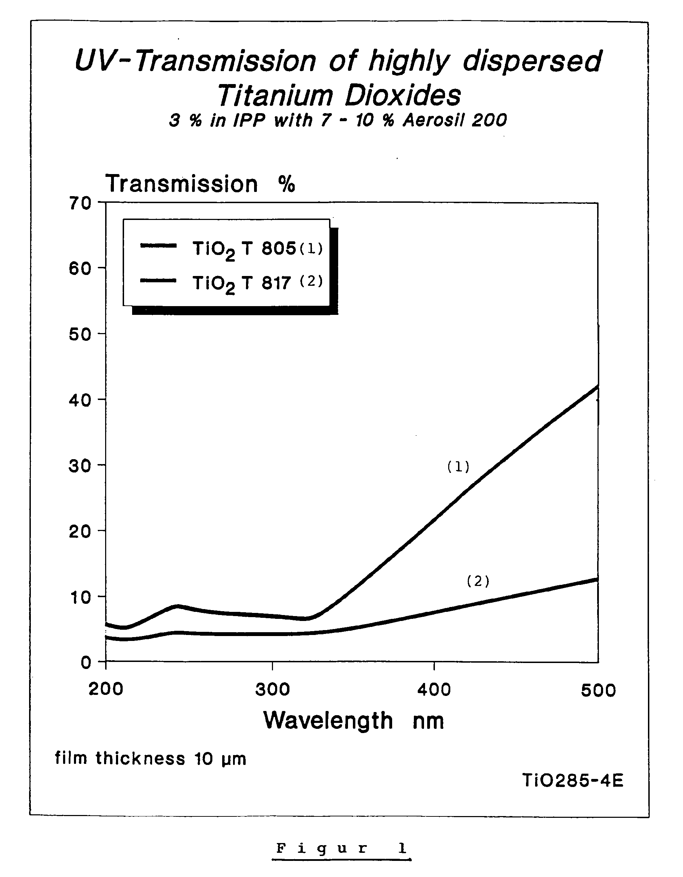

In der in der Figur 1 aufgeführten Absorptionskurve ist das Produkt aus Beispiel 13 (VT 817, Fe-Ti-Mischoxid nachbehandelt mit Si 108 = Trimethoxyoctylsilan) dem Produkt T 805 (pyrogenes TiO₂ nachbehandelt mit Si 108 = Trimethoxyoctylsilan) gegenübergestellt. Wie man erkennen kann, zeigt T 817 eine deutlich stärkere Absorption insbesondere im UV-A-Bereich, was einen Vorteil in der Anwendung in Sonnenschutzmitteln darstellt.

Die Absorpitonskurve wurde wie folgt ermittelt:

Die Oxide wurden 3 %ig in Isopropylpalmitat dispergiert. Mit Hilfe von Aerosil 200 wurden die Dispersionen stabilisiert. Anschließend wurde die Transmission zwischen 200 und 500 nm in einer Schichtdicke von 10 µm gemessen.

## Patentansprüche

1. Oberflächenmodifizierte pyrogen hergestellte Mischoxide, die zwei oder mehr Komponenten aus der Reihe SiO₂, Al₂O₃, TiO₂, ZrO₂, Fe₂O₃, Nb₂O₅, V₂O₅, WO₃, SnO₂, GeO₂ enthalten und die von anhaftenden Chlorwasserstoff in feuchter Luft befreit wurden und mit einer oder mehreren Verbindungen aus den folgenden Gruppen oberflächenmodifiziert wurden:
(a) Organosilane des Types (RO)₃Si(CₙH₂ₙ₊₁)R = Alkyl
n = 1 - 20
(b) Organosilane des Types R'ₓ(RO)_{y}Si(CₙH₂ₙ₊₁) R = Alkyl
R' = Alkyl
n = 1 - 20
x+y = 3
x = 1,2
y = 1,2
(f) Organosilane des Types (RO)₃Si(CH₂)ₘ-R'
R =Alkyl
m = 0,1 - 20
R' = Methyl-, Aryl (z.B. -C₆H₅, substituierte Pheneylreste) -C₄F₉, OCF₂-CHF-CF₃, -C₆F₁₃, -O-CF₂-CHF₂ -NH₂, -N₃, -SCN, -CH=CH₂, -OOC(CH₃)C = CH₂ -OCH₂-CH(O)CH₂ -NH-CO-N-CO-(CH₂)₅ 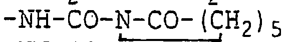 -NH-COO-CH₃, -NH-COO-CH₂-CH₃, -NH-(CH₂)₃Si(OR)₃ -Sₓ-(CH₂)₃Si(OR)₃
(g) Organosilane des Typs (R'')ₓ(RO)_{y}Si(CH₂)ₘ-R'
R'' Alkyl x+y = 3
x = 1,2
y = 1,2
m = 0,1 bis 20
R' = Methyl-, Aryl (z.B. -C₆H₅, substituierte Phenylreste) -C₄F₉, -OCF₂-CHF-CF₃, -C₆F₁₃, -O-CF₂-CHF₂ -NH₂, -N₃, -SCN, -CH=CH₂, -OOC(CH₃)C = CH₂ -OCH₂-CH(O)CH₂ 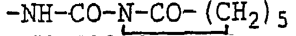 -NH-COO-CH₃, -NH-COO-CH₂-CH₃, -NH-(CH₂)₃Si(OR)₃ -Sₓ-(CH₂)₃Si(OR)₃
R = Methyl-, Ethyl-
(k) Silazane des Types 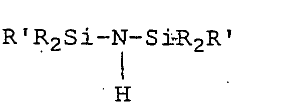 K = Alkyl
R' = Alkyl, Vinyl
(m) Polysiloxane bzw. Silikonöle des Types
m = 0, 1, 2, 3, ...∞
n = 0, 1, 2, 3, ...∞
u = 0, 1, 2, 3, ...∞
Y=CH₃, H, CₙH₂ₙ₊₁ n=1-20
Y=Si(CH₃)₃, Si(CH₃)₂H
Si (CH₃)₂OH, Si(CH₃)₂(OCH₃)
Si(CH₃)₂(CₙH₂ₙ₊₁) n=1-20
R = Alkyl, Aryl. (CH₂)ₙ - NH₂, H
R' = Alkyl, Aryl, (CH₂)ₙ - NH₂, H
R'' = Alkyl, Aryl, (CH₂)ₙ - NH₂, H
R''' = Alkyl, Aryl, (CH₂)ₙ - NH₂, H

2. Verfahren zur Herstellung von oberflächenmodifizierten pyrogen hergestellten Mischoxiden nach Anspruch 1, **dadurch gekennzeichnet, daß** man zwei oder mehr Metallchloride aus der Reihe SiCl₄, AlCl₃, TiCl₄, ZrCl₄, FeCl₃, NbCl₅, VOCl₃, WOCl₄, WCl₆, SnCl₄ und GeCl₄ gemeinsam oder getrennt verdampft, zusammen mit einem Inertgas, zum Beispiel Stickstoff, in die Mischkammer eines bekannten Brenners überführt, dort mit Wasserstoff, Luft und/oder Sauerstoff vermischt, das Mehrkomponentengemisch in einer Reaktionskammer verbrennt, danach die festen Mischoxide von den gasförmigen Reaktionsprodukten abtrennt und in feuchter Luft von anhaftendem Chlorwasserstoff befreit und die pyrogen hergestellten Mischoxide in einem geeigneten Mischgefäß vorlegt, unter intensivem Mischen die Mischoxide gegebenenfalls zunächst mit Wasser und anschließend mit dem Oberflächenmodifizierungsreagenz oder dem Gemisch von mehreren Oberflächenmodifizierungsreagentien besprüht, 15 bis 30 Minuten nachmischt und anschließend bei einer Temperatur von 100 bis 400 °C über einen Zeitraum von 1 bis 6 Stunden tempert.

3. Verwendung des oberflächenmodifizierten pyrogen hergestellten Mischoxides nach Anspruch 1 als
- Verstärkerfüllstoff in Silikonkautschuk und Gummi
- Ladungsstabilisator und Rieselhilfsmittel in 5 Tonerpulver
- Rieselhilfsmittel (free-flow-agent)
- Antiblockingmittel, zum Beispiel in Folien
- UV-Blocker, zum Beispiel in Kosmetika
- Verdickungsmittel, zum Beispiel in Lacken

## Claims

1. Surface-modified, pyrogenically produced mixed oxides which contain two or more components from the series consisting of SiO₂, Al₂O₃, TiO₂, ZrO₂, Fe₂O₃, Nb₂O₅, V₂O₅, WO₃, SnO₂, GeO₂ and which have been freed from adhering hydrogen chloride in moist air and have been surface-modified with one or more compounds of the following groups:
(a) organosilanes of the formula (RO)₃Si(CₙH₂ₙ₊₁) R = alkyl
n = 1-20
(b) organosilanes of the formula R'ₓ(RO)_{y}Si(CₙH₂ₙ₊₁)R = alkyl
R' = alkyl
n = 1-20
x+y = 3
x= 1, 2
y = 1, 2
(c) organosilanes of the formula (RO)₃Si(CH₂)ₘ-R'
R = alkyl
m = 0, 1-20
R' = methyl, aryl (e.g. -C₆H₅, substituted phenyl radicals),
-C₄F₉, OCF₂-CHF-CF₃, -C₆F₁₃, O-CF₂-CHF₂
-NH₂, -N₃, -SCN, -CH=CH₂,
-OOC(CH₃) C = CH₂
-OCH₂-CH (O) CH₂
-NH-CO-N-CO-(CH₂)₅
-NH-COO-CH₃, -NH-COO-CH₂-CH₃, -NH-(CH₂)₃Si(OR)₃
-Sₓ- (CH₂)₃Si (OR)₃
(d) organosilanes of the formula (R")ₓ(RO)_{y}Si(CH₂)ₘ-R'
R" = alkyl x+y = 3
x = 1, 2
y = 1, 2
m = 0, 1 to 20
R' = methyl, aryl (e.g. -C₆H₅, substituted phenyl radicals)
-C₄F₉, -OCF₂-CHF-CF₃, -C₆F₁₃, -O-CF₂-CHF₂
-NH₂, -N₃, -SCN, -CH=CH₂,
-OOC(CH₃) C = CH₂
-OCH₂-CH(O)CH₂
-NH-CO-N-CO- (CH₂)₅
-NH-COO-CH₃, -NH-COO-CH₂-CH₃, -NH-(CH₂)₃Si(OR)₃
-Sₓ-(CH₂)₃Si(OR)₃
R = methyl, ethyl,
(e) silazanes of the formula 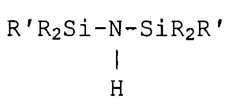 R = alkyl
R' = alkyl, vinyl,
(f) polysiloxanes or silicone oils of the formula 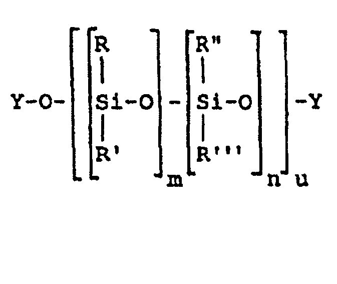 m = 0, 1, 2, 3, ... ∞
n = 0, 1, 2, 3, ... ∞
u = 0, 1, 2, 3, ... ∞
Y=CH₃, H, CₙH₂₊₁ n=1-20
Y=Si(CH₃)₃, Si (CH₃)₂H
Si(CH₃)₂OH, Si(CH₃)₂(OCH₃)
Si (CH₃)₂(CₙH₂ₙ₊₁) n=1-20
R = alkyl, aryl, (CH₂)ₙ - NH₂, H
R' = alkyl, aryl, (CH₂)ₙ - NH₂, H
R'' = alkyl, aryl, (CH₂)ₙ - NH₂, H
R''' = alkyl, aryl, (CH₂)ₙ - NH₂, H

2. Process for preparing surface-modified, pyrogenically produced mixed oxides according to Claim 1, **characterized in that** two or more metal chlorides selected from the series consisting of SiCl₄, AlCl₃, TiCl₄, ZrCl₄, FeCl₃, NbCl₅, VOCl₃, WOCl₄, WCl₆, SnCl₄ and GeCl₄ are evaporated together or separately, transferred together with an inert gas, for example nitrogen, into the mixing chamber of a known burner, mixed there with hydrogen, air and/or oxygen, the multi-component mixture is burned in a reaction chamber, the solid mixed oxides are then separated from the gaseous reaction products and freed from adhering hydrogen chloride in moist air, and the pyrogenically produced mixed oxides are charged to a suitable mixing vessel, the mixed oxides are sprayed, with intensive mixing, optionally first with water and then with the surface modifying reagent or the mixture of a plurality of surface-modifying reagents, mixed again for 15 to 30 minutes and subsequently heat-treated at a temperature of 100 to 400°C over a period of 1 to 6 hours.

3. Use of the surface-modified, pyrogenically produced mixed oxide according to Claim 1 as
- a reinforcing filler in silicone rubber
- a charge stabilizer and flow aid in toner powder
- a free-flow agent
- an antiblocking agent, for example in films
- a UV blocker, for example in cosmetics
- a thickener, for example in coatings.

## Revendications

1. Oxydes mixtes modifiés en surface et fabriqués par pyrogénation qui contiennent deux composants ou plus de la série des SiO₂, Al₂O₃, TiO₂, ZrO₂, Fe₂O₃, Nb₂O₅, V₂O₅, WO₃, SnO₂, GeO₂ et qui sont libérés dans l'air humide du chlorure d'hydrogène adhérant et sont modifiés en surface avec un ou plusieurs composés des groupes suivants:
(a) organosilanes du type (RO)₃Si(CₙH₂ₙ₊₁) R=alkyle
n=1-20
(b) organosilanes du type R'ₓ(RO)_{y}Si(CₙH₂ₙ₊₁) R=alkyle
R'=alkyle
n=1-20
x+y=3
x=1,2
y=1,2
(c) organosilanes du type (RO)₃Si(CH₂)ₘ-R'
R=alkyle
M=0,1-20
R'= méthyl-, aryle (par exemple -C₆H₅, radical phényle substitué)
-C₄F₉, OCF₂-CHF-CF₃, -C₆F₁₃, -O-CF₂-CHF₂
-NH₂, -N₃, -SCN, -CH=CH₂,
-OOC(CH₃)C = CH₂
-OCH₂-CH (O)CH₂
-NH-CO-N-CO-(CH₂)₅
-NH-COO-CH₃, -NH-COO-CH₂-CH₃, -NH- 5CH₂)₃Si(OR)₃
-Sₓ-(CH₂)₃Si(OR)₃
(d) organosilanes du type (R")ₓ(RO)_{y}Si(CH₂)ₘ-R'
R"=alkyle x+y=3
x=1,2
y=1,2
m=0,1 à 20
R'=méthyl-, aryle (par exemple -C₆H₅, radical phényle substitué)
-C₄F₉, -OCF₂-CHF-CF₃, -C₆F₁₃, -O-CF₂-CHF₂
-NH₂, -N₃, -SCN, -CH=CH₂,
-OOC(CH₃)C = CH₂
-OCH₂-CH(O)CH₂ 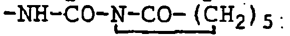 -NH-COO-CH₃, -NH-COO-CH₂-CH₃, -NH-(CH₂)₃Si(OR)₃
-Sₓ-(CH₂)₃Si(OR)₃
R=méthyl-, éthyl-
(e) silazanes du type 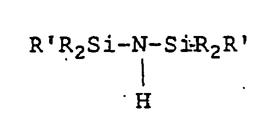 R=alkyle
R'=alkyle, vinyle
(f) polysiloxanes ou huiles de silicone du type 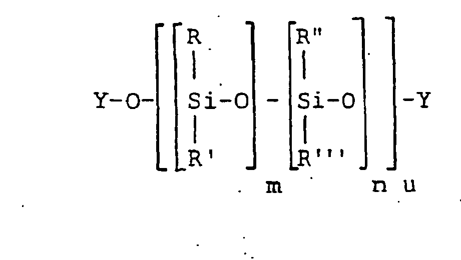 m = 0,1,2,3;...∞
n = 0,1,2,3,...∞
u = 0,1,2,3,...∞
Y=CH₃, H, CₙH₂ₙ₊₁ n=1-20
Y=Si(CH₃)₃, Si(CH₃)₂H
Si(CH₃)₂OH, Si(CH₃)₂(OCH₃)
Si(CH₃)₂(CₙH₂ₙ₊₁) n=1-20
R = Alkyle, Aryle, (CH₂)ₙ - NH₂, H
R' = Alkyle, Aryle, (CH₂)ₙ - NH₂, H
R'' = Alkyle, Aryle, (CH₂)ₙ - NH₂, H
R''' = Alkyle, Aryle, (CH₂)ₙ - NH₂, H

2. Procédé de fabrication d'oxydes mixtes modifiés en surface et fabriqués par pyrogénation selon la revendication 1, **caractérisé en ce qu'**on évapore ensemble ou séparément deux chlorures de métal ou plus de la série des SiCl₄, AlCl₃, TiCl₄, ZrCl₄, FeCl₃, NbCl₅, VOCl₃, WOCl₉, WCl₆, SnCl₄ et GeCl₄, avec un gaz inerte, par exemple de l'azote, on les transfère dans la chambre de mélange d'un brûleur connu, on les y mélange avec de l'hydrogène, de l'air et/ou de l'oxygène, on calcine le mélange à plusieurs composants dans une chambre de réaction, on sépare ensuite les oxydes mixtes solides des produits réactionnels gazeux et on les libère dans de l'air humide du chlorure d'hydrogène adhérant et on dépose les oxydes mixtes fabriqués par pyrogénation dans un récipient mélangeur approprié, on pulvérise sous mélange intensif les oxydes mixtes le cas échéant d'abord avec de l'eau et ensuite avec le réactif de modification de surface ou avec le mélange de plusieurs réactifs de modification de surface, on mélange encore pendant 15 à 30 minutes et ensuite on porte à une température de 100 à 400°C sur une durée de 1 à 6 heures.

3. Utilisation de l'oxyde mixte modifié en surface et fabriqué par pyrogénation selon la revendication 1 comme:
- charge de renforcement dans le caoutchouc de silicone et la gomme
- stabilisant de charge et agent de ruissellement dans la poudre toner
- agent de ruissellement (free-flow-agent)
- agent anti-blocage, par exemple dans des feuilles
- bloqueur d'UV, par exemple dans la cosmétique
- épaississant, par exemple dans les peintures.
